## Europäisches Patentamt

## European Patent Office

## Office européen des brevets

(11) Publication number: **0 051 935**

**B1**

(12) **EUROPEAN PATENT SPECIFICATION**

(45) Date of publication of patent specification: **20.11.86**

(51) Int. Cl.⁴: **A 61 F 13/02, A 61 B 19/08**

(21) Application number: **81304905.3**

(22) Date of filing: **20.10.81**

(54) Medical device for covering a portion of the anatomical surfaces of a living being.

(30) Priority: **10.11.80 US 205344**

(43) Date of publication of application:
**19.05.82 Bulletin 82/20**

(45) Publication of the grant of the patent:
**20.11.86 Bulletin 86/47**

(84) Designated Contracting States:
**CH DE FR GB IT LI NL SE**

(56) References cited:
**DE-A-1 935 916**
**US-A-2 565 509**
**US-A-2 924 331**
**US-A-2 927 689**
**US-A-3 065 120**
**US-A-4 161 176**

(73) Proprietor: **MINNESOTA MINING AND MANUFACTURING COMPANY**
**3M Center, P.O. Box 33427**
**St. Paul, Minnesota 55133-3427 (US)**

(72) Inventor: **Heinecke, Steven B.**
**2501 Hudson Road P.O. Box 33427**
**St. Paul Minnesota 55133 (US)**

(74) Representative: **Baillie, Iain Cameron et al**
**c/o Ladas & Parry Isartorplatz 5**
**D-8000 München 2 (DE)**

Courier Press, Leamington Spa, England.

**Description**

This invention relates to a medical device having a conformable, thin polymeric adhesive-coated film combined with a release liner and releasable layer to allow easy application of the films to the body.

In the medical art, thin, conformable polymeric films which are coated with pressure-sensitive adhesives are sometimes used as wound dressings and surgical drapes. Polyurethane films which are adhesively coated on one surface are presently being sold for use on the body such as for attaching catheters to the body and as wound dressings. As sold, the film is attached to a release liner with one portion of the film forming a tab and being non-tacky. These films, particularly those of larger sizes, are very difficult to place on the body because when they are removed from the release liner they tend to block or resiliently stick to themselves and thus are an unwieldy product. Additionally, a thin, conformable resilient film which is applied by use of a tab on the film often places the substrate under the film after application under excessive compression because of the resiliency of the film. U.S. Patent 3,645,835 discusses polyurethane films coated with pressure-sensitive adhesives for use on the body. Surgical drapes containing tab portions for placement on the body are disclosed in U.S. Patent 3,260,260.

Prior to applicant's invention a need existed in the medical art for a device and method for applying pressure-sensitive adhesive coated thin, polymeric films to the body which eliminated or significantly reduced the tendency of the films to block or otherwise become wrinkled and stick to themselves, which reduced or eliminated compression of the substrate, and which allowed placement of the film on the body without touching the adhesive surface of the film. Previous attempts to provide a means to apply a bandage containing a polymeric film or sheet have involved the use of an adhesive located on the side of the bandage opposite to the pressure-sensitive adhesive to be attached to the skin. See for example, U.S. Patents 3,520,306; 2,752,038; 2,924;331. Other means of handling bandages are described in U.S. Patents 2,703,083; 2,927,689; 3,007,571 and 4,182,449. None of these have involved Applicant's device amd method for application of pressure-sensitive adhesive-coated thin, conformable polymeric films to a substrate.

Handling thin layers of materials has been discussed in the decal art. For example in U.S. Patent 4,028,474 a double-backed decal is described which involves holding the backing in place with two different adhesives. In U.S. Patent 3,065,120, another decal patent, a polyamide release sheet is to be used on the surface of the decal opposite to that to which the tacky adhesive is attached, the tacky adhesive being covered with a releasable sheet of paper. The polyamide release sheet contacts a clear layer of relatively stiff vinyl lacquer and this vinyl lacquer layer is to have interfacial tension with the polyamide surface so that it remains temporarily attached thereto. The vinyl lacquer is described as being selected from the group consisting of cellulose esters and ethers, acrylic polymers and vinyl chloride and acetate polymers and copolymers. After the decal is positioned by removing the adhesive covering slip sheet of paper and placing the adhesive on the substrate the polyamide surface paper is removed. Neither of these disclosures in the decal art deal with the problem of handling thin, conformable polymeric adhesive-coated films as herein described.

German Offenlegungsschrift 1935916 discloses a roll of film bandaging material comprising a relatively thin polymeric film which is conformable to anatomical surfaces of living beings, a pressure sensitive adhesive covering one major surface of the film and a carrier substrate with a release coating attached to the other major surface of the film. The carrier substrate assists in handling the film and applying it to an anatomical surface and is to be peeled off after the film is in place on the anatomical surface.

According to the present invention there is provided a medical device for covering a portion of the anatomical surfaces of a living being comprising: (a) a relatively thin polymeric film having an upper surface and a lower surface, said film being conformable to said portion of said anatomical surfaces; (b) a pressure sensitive adhesive covering at least a portion of said upper surface of said film; (c) a release liner attached to said adhesive-covered upper surface of said film and; (d) a releasable layer attached to said lower surface of said film, characterized in that said releasable layer comprises a frame attached to at least a major portion of the periphery of said lower surface of said film to support said film in a flat, wrinkle-free condition until adhered to said portion of said anatomical surfaces, said releasable layer having an opening in the central portion thereof exposing a substantial portion of said film, and said releasable layer being attached to said lower surface of said film more tenaciously than the release liner is attached to said adhesive-covered upper surface of said film. Optional features of the invention are referred to in claims 2—12.

In the preferred embodiment of the invention, releasable layer attached to said lower surface of said film is divided into at least two parts which may be removed from said film independently from each other, at least one of said parts overlying the central portion of said film so that when removed from said film, a major portion of said film is exposed, and at least one of said parts overlying a substantial portion of the periphery of said film so that when the part of said releasable layer overlying the central portion of said film is removed first, the part of said releasable layer overlying the periphery of said film provides the frame to support said film in a flat, wrinkle-free condition until adhered to said portion of said anatomical surfaces.

Of particular importance is the use of the device in the medical field wherein thin, conformable polymeric adhesive-coated films are applied to the skin by a single operator and oftentimes while an attempt is being made to use the film to hold in place some other device or instrument. Thus it is important

2

that only a single hand be needed for placement of the film and that the film not block or otherwise stick together. The device provides this capability. Additionally, the device, as will be discussed below, allows placement of the film without significant compression of the tissue by the resilient forces of the film and without contact by the operator with the adhesive coated side of the film in the embodiments when a tab is present. Preferably the releasable layer of the device is at least translucent to permit visual observation therethrough of the substrate to which the film is being applied. A specific device embodying the invention will be discussed in more detail with reference to the following drawings in which:

Figure 1 is an expanded perspective view showing one embodiment of the device of the present invention,

Figure 2 is a perspective view of the device of Figure 1,

Figure 3 is a view of the device of Figures 1 and 2 being applied to the body limb,

Figure 4 is a section taken along line 5 of Figure 3, and

Figure 5 is a view of the device of Figure 3 after placement is complete on the body.

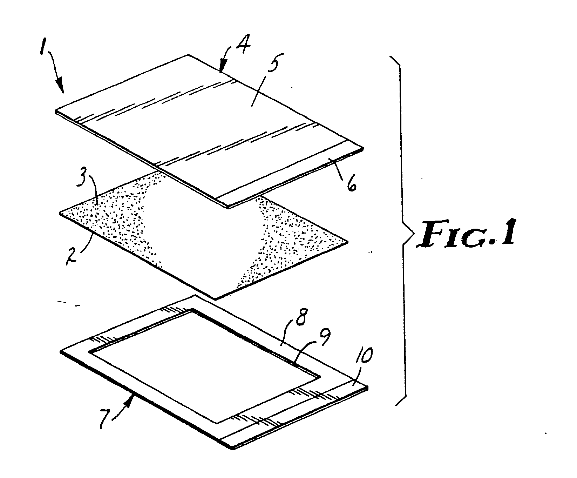

Referring to the figures in more detail, Figure 1 discloses the device 1 comprising thin, conformable polymeric film 2 coated with pressure-sensitive adhesive 3 on the upper surface thereof. Above but normally attached to the upper surface of film 2 containing adhesive 3 is release liner 4 containing portion 5 for covering of adhesive 3 and tab 6 which provides a means for removal of the release liner 4 from adhesive 3 without touching and thus contaminating adhesive 3. Below film 2 is releasable layer 7 comprising frame 8 surrounding opening or perforation 9 and containing tab portion 10 for purposes of removal of releasable layer 7 from film 2 without touching film 2. Tab 6 and tab portion 10 are optional but are preferred for the aforesaid reasons. Tab 6 and tab portion 10 can be integrated with release liner 4 and frame 8, respectively or can be attached separately by adhesive or other well known means. Releasable layer 7 is not attached to film 2 by a pressure-sensitive adhesive but is normally attached to the film 2 by means of the mechanical attachment forces which result when film 2 is extruded or otherwise formed on releasable layer 7. Releasable layer 7, at the time of formation, does not include perforation or opening 9 as will be described in more detail later along with the composition and construction of release liner 4, film 2, adhesive 3 and releasable layer 7. Perforation 9 provides frame 8 with flexibility and conformability.

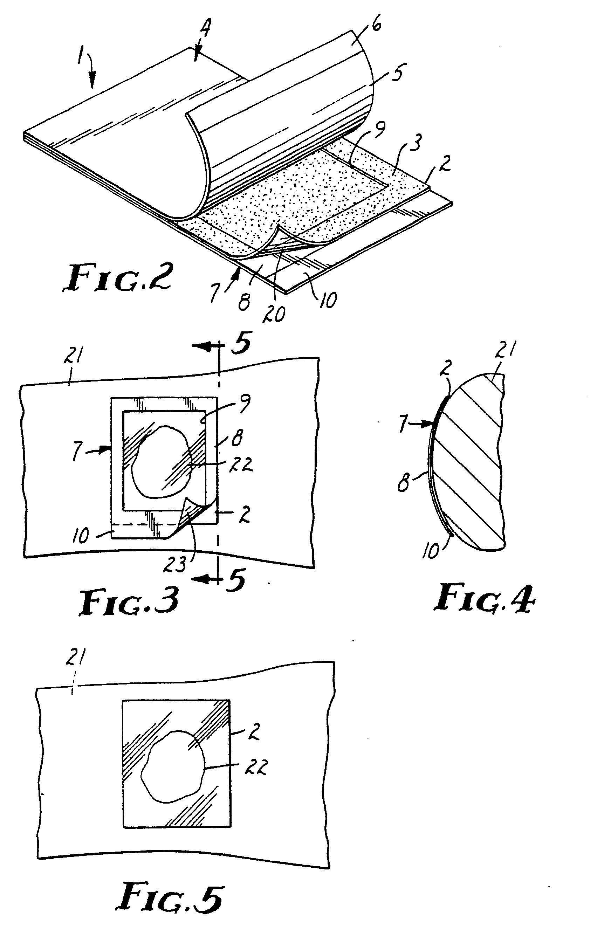

Figure 2 depicts device 1 with the parts thereof shown attached to each other, specifically release liner 4 is attached at portion 5 to the adhesive 3 of film 2. The tab 6 of release liner 4 has been pulled so that a portion of adhesive 3 on film 2 is exposed. This depicts how release liner 4 is removed from film 2 containing adhesive 3, i.e., tab 6 is grasped as well as tab 10 of releasable layer 7. Since the release liner 4 is removed more easily from adhesive 3 than is releasable layer 7 from film 2, the release liner 4 is removed leaving film 2 containing adhesive 3 with releasable layer 7 containing tab portion 10, opening 9 and frame 8 attached thereto. In Figure 2 a corner 20 of film 2 is raised to show how releasable layer 7 is removed from film 2 and to indicate that there is no adhesive attaching film 2 to releasable layer 7. Also in Figure 2 opening 9 is visible because film 2 and adhesive 3 are transparent.

Figure 3 depicts the placement of device 1 on a human body limb 21. In Figure 3 device 1 has removed from it release liner 4, thus release liner 4 is not shown. Film 2 containing adhesive 3 (not shown) is now adhesively adhered to limb 21 over wound 22. When film 2 was placed on limb 21 over wound 22 it remained attached to releasable layer 7 containing frame 8, opening 9 and tab 10. Layer 7 provides the means by which the film can be handled without blocking or otherwise becoming wrinkled and sticking to itself. It is a flat continuous wrinkle-free film until placed on the skin and releasable layer 7 is removed. The importance of opening 9 in releasable layer 7 and thus the form of frame 8 being what it is, i.e. a frame, is graphically shown in Figure 3. Film 2 is transparent and opening 9 in combination with transparent film 2 provides a visual observation of wound 22 and an accurate placement of film 2 over wound 22. Figure 3 depicts corner 23 of releasable layer 7 being partially pulled away from film 2. This indicates how releasable layer 7 is removed from film 2 when film 2 is in place and adhesively attached to limb 21. The adhesive forces holding film 2 on limb 21 are greater than the forces holding releasable layer 7 on film 2. Generally the adhesion to skin determined as described later is greater than about 40 gm per 2.54 centimeters width. Thus the releasable layer 7 is removed from film 2 when tab 10 is pulled rather than film 2 being pulled from limb 21.

Figure 4, which is a partial section view of limb 21 taken along line 5 of Figure 3, shows releasable layer 7 overlying film 2 which is attached to limb 21 and also indicates that tab 10 of releasable layer 7 extends beyond film 2 and provides an easy means to grasp releasable layer 7 and remove it from film 2. Frame 8 of releasable layer 7 can contain a slit rather than tab 10 to provide a means of removing the frame 8 from film 2.

Figure 5 depicts film 2 in place and adhesively attached over wound 22 on limb 21 with releasable layer 7 (not shown) removed therefrom.

Film 2 of the device described herein is a thin, conformable polymeric film. Generally the film is from 12 to 50 microns in thickness, preferably from 12 to 25 microns. Conformability is somewhat dependent on thickness, thus the thinner the film the more conformable the film. Reference has been made herein to the films utilized in the device being conformable to animal anatomical surfaces. This means that when the films are applied to an animal anatomical surface it conforms to the surface even when the surface is moved. The preferred films are conformable to animal anatomical joints. When the joint is flexed and then returned to its unflexed position, the film stretches to accommodate the flexation of the joint but is resilient enough to continue to conform to the joint when the joint is returned to its unflexed condition. A measure of conformability is the $F_{10}$ modulus of the film which is the pounds (grams) force it takes to stretch a material ten percent of its original length. The films preferably have a $F_{10}$ modulus no greater than about 454 grams (1 pound) and preferably less than about 363 grams (0.8 pounds). The device can be utilized with films which have $F_{10}$ moduli upwards of 1135 grams (2.5 pounds), however, as the $F_{10}$ modulus increases the conformability decreases and the ability to handle the films without the previously discussed blocking problems is increased.

$F_{10}$ modulus as referred to herein is determined using an Instron Unit Model 1102 from Instron Corp., 2500: Washington Street, Canton, Massachusetts. The cross-head speed of the Instron is 25.4 cm (ten inches) per minute and the chart speed is set at 25.4 cm (ten inches) per minute. The gauge length is set at 5 cm (two inches) with the test sample cut to test a 2.54 cm (one-inch) width.

Examples of films which are useful in Applicant's invention include polyurethane, elastomeric polyester such as DuPont "Hytrel" (TM) polyester elastomer (Wilmington, Delaware), polyethylene, blends of polyurethane and polyester, chlorinated polyethylene, styrene/butadiene block copolymers such as "Kraton" (TM) brand thermoplastic rubber (Shell Chemical Company, Houston, Texas), and polyvinyl chloride. Particularly preferred films are polyurethane and elastomeric polyester films. The polyurethane and elastomeric polyester films exhibit a resilient property which allows the film to have good conformability. However, this property also causes them to compress the tissue if the film is applied under tension onto the wound site, i.e., if the film is in a stretched condition when it is placed on the wound. Thus, the device is particularly useful with polyurethane films and other films which exhibit a significant degree of resiliency.

For use on animal bodies, it is preferred that the film with the adhesive attached be moisture vapor permeable. Certain polyurthane films, elastomeric polyester films and blends of polyester and polyurethane films are moisture vapor permeable. Other films, such as chlorinated polyethylene and polyethylene, are not significantly moisture vapor permeable in an unperforated form. The films are preferably at least translucent and more preferably transparent so that if a translucent or transparent releasable layer is used in the device the wound site or substrate to which the film is to be applied can be viewed through the releasable layer as well as the film when the film is being applied to the substrate.

The preferred adhesives which can be used in the device are the normal adhesives which are applied to the skin such as those described in Ulrich U.S. Patent RE 24,906, particularly a copolymer of 96% iso-octyl acrylate units and 4% acrylamide units and a copolymer of 94% iso-octyl acrylate units and 6% acrylic acid units. Other useful adhesives are those described in U.S. Patent 3,389,827 which comprise block copolymers having three or more polymer block structures having a general configuration --A -- B -- A --- wherein each A is a thermoplastic polymer block with a glass transition temperature above room temperature (i.e., above about 20°C.) having an average molecular weight between about 5000 and 125,000 and B is a polymer block of a conjugated diene having an average molecular weight between about 15,000 and 250,000. Additional examples of useful adhesives are iso-octyl acrylate/n-vinyl pyrrolidone copolymer adhesives and crosslinked acrylate adhesives such as for example those described in U.S. Patent 4,112,213. Inclusion in the adhesive of medicaments or antimicrobial agents such as iodine is useful for enhancing wound healing and preventing infection.

· The release liner which is attached to the adhesive on the film is a liner which releases with less force than is required for the releasable layer to be removed from the film. Generally the adhesive to liner as determined in accordance with ASTM D3330—76 is between about 3 and 20 grams per 2.54 cm width while the adhesion to releasable layer of the film is greater than that to the liner and ranges up to about 70 grams per 2.54 cm width. Examples of release liners are liners made of or coated with polyethylene, polypropylene and fluorocarbons and silicone coated release papers or polyester films. Examples of the silicone coated release papers are Polyslik S-8004 (TM), 37682 grams (83 pound) bleached silicone release paper supplied by H. P. Smith Co., Chicago, Illinois, and 36320 grams (80 pound) bleached two-sided silicone coated paper supplied by Daubert Chemical Co., Dixon, Illinois. Releasable layer 7 of device 1 comprises a material which will adhere to the film 2 with a greater tenacity than release liner 4 adheres to adhesive 3 in order that the release liner is removed prior to the removal of the releasable layer. The releasable layer can comprise materials generally of the type described in respect to the release liner although as noted above more adherent varieties or surfaces of the above materials will be used as a releasable layer. The releasable layer is attached with less tenacity to the film than the adhesive attaches the film to the substrate such as the skin.

Device 1 is manufactured using conventional film forming techniques, for example extrusion, casting or calendaring as well as conventional adhesive placement and slitting techniques. The releasable layer is preferably coated with the film by means of extruding the polymeric substance through a die onto the releasable layer. Adhesive is then applied to the film using normal direct or transfer coating techniques. The film is cut to allow for the tabs using control depth cutting techniques. The release liner is then placed over the adhesive of the film and the combination is appropriately die cut, by control-depth die cutting to provide the final product. Normally the portions of the releasable layer which is cut to provide the perforation 9 in frame 8 of Figure 1 will be retained in the perforation until it is removed by the user prior to use. It should be noted that although the portion of the releasable layer 7 of Figure 1 which is removed to form perforation 9 is attached more tenaciously to the film 2 than the release liner 4 is to adhesive 3, the

4

portion of releasable layer 7 to be removed can be removed without removing the release liner because the device 1 is grasped at the tabs 6 and 10 or elsewhere to permit said removal. In some instances formation of perforation 9 by removal of a portion of releasable layer 7 will cause the film 2 to be pulled in small, insignificant areas from release liner 4.

The device is produced in the form of individual units having the configuration shown in, for example, Figure 1. Alternatively the device is packaged as a continuous roll of adhesive coated film with a releasable layer attached to the non-adhesive surface of the film. In roll form, one surface of the releasable layer is attached to the non-adhesive surface of the film and the adhesive layer of the film is rolled onto the continuous length of the combination so that the adhesive surface contacts the opposite surface of the releasable layer to that attached to the non-adhesive surface of the film. In this configuration the releasable layer and release liner are one and the same. However, the releasable layer is attached more tenaciously to the film than the adhesive is attached to the releasable layer surface which is adjacent the adhesive. Alternatively, a separate release liner could be used with the roll form of the device.

It is contemplated that additional appliances or materials can be attached to the non-adhesive surface of the film, such as by attaching a colostomy bag to the non-adhesive surface of the film through the perforation 9 in frame 8 in the embodiment shown in Figure 1. Also, absorbent material such as gauze or other compresses could be attached to the adhesive surface layer of the film for application to a wound site.

The following examples are meant to illustrate but not to limit the invention. In the examples adhesion to liner and adhesion to releasable layer values were determined in accordance with ASTM D3330—76. It should be noted that the adhesion to skin data is not comparable with the adhesion to liner and adhesion to releasable layer data because a different test procedure is used. The adhesion to liner and adhesion to releasable layer data is comparable. Adhesion to skin was determined in accordance with the procedure set forth hereinafter. The adhesive properties are determined on human skin since there is no *in vitro* model which correlates well with human skin.

The adhesion to skin was measured as follows:

1. Adhesive coated films 2.54 cm. wide by approximately 7.62 cm. long are placed on the back of a human subject.

2. Each film is rolled down with one forward and one reverse pass of a 1 kg. tape roller moved at a rate of about 30 cm. per minute. The roller used is of the type described in *Test Methods for Pressure-Sensitive Tapes* (Pressure-Sensitive Tape Council, Glenview, Ill.) Appendix B, Sections 2.7.1; 2.8.1; and 2.8.2.

3. Adhesion to skin is measured by 180 degrees peel-type removal. The peel force values are measured through the use of a strain-gauge mounted on a motor-driven carriage. The force of the removal is reported in grams of adhesion per 2.54 cm. width of sample. The rate of removal is 15 cm. per minute.

4. The adhesion to skin is measured immediately after placement on the skin.

Example 1

A 25 micron (one mil) film of "Estane" (TM) 5707—F1 polyurethane resin (B. F. Goodrich, Cleveland Ohio) was extruded using a 1.9 cm (three-quarter inch) Rheomex Model 252 screw extruder (manufactured by Haake, Saddlebrook, New Jersey), a sheeting die and a melt temperature of 200°C. The film was extruded onto the back side of a clay-coated side of a 35412 grams (78 pound) paper (releasable layer) which was clay-coated on one side by roll coating (No. 70—05—04—000, Boise Cascade Corporation, International Falls, Minnesota). Immediately after extrusion the paper/resin combination was passed through a nip roll at 2812 grams per square centimeter (40 psi). Twenty-five grams per square meter of an adhesive prepared in accordance with U.S. Patent Reissue 24,906 comprising a copolymer of 96% units of isooctylacrylate and 4% units acrylamide was applied to the surface of the film that was not attached to the clay-coated paper utilizing a standard horizontal knife lab coater. A release liner comprising 36320 grams (80 pound) bleached, two side coated, silicone paper (2—80 BKG-157, Daubert Chemical Company, Dixon, Illinois) was applied to the adhesive of the film. Samples of one lot of the composite were cut for purposes of obtaining adhesion to liner, initial adhesion to skin and adhesion to releasable layer data. In addition, samples of another lot were prepared of the configurations shown in Figures 1 using a die-cutting machine for control depth die cutting (Model 813, Series K7Y223, Mark Andy, St. Louis, Missouri) and a razor blade. The samples were tested both sterilized and nonsterile. Sterilization was obtained using three megarads of gamma radiation.

Average grams per 2.54 cm width

|  | Adhesion to liner | Initial adhesion to skin 18 subjects | Adhesion to releasable layer |
|---|---|---|---|
| Non-Sterile | 4.3 | 50.56 | 50 |
| Sterile | 8.3 | 57.57 | 66.7 |

The $F_{10}$ modulus of the film was 250 grams (0.55 pounds).

# 0 051 935

Example 2

The procedure of Example 1 was followed except that the adhesive used was a polymer of 96% units of isooctylacrylate and 4% units acrylic acid and the amount of adhesive applied was 40 grams per square meter. Results were as follows: Average adhesion to liner, 16 grams per 2.54 cm. width; average initial adhesion to skin (6 subjects), 99.11 grams per 2.54 cm. width and adhesion to releasable layer, 27 grams per 2.54 cm. width. The $F_{10}$ modulus of the film was again 25 gm (0.55 pound).

Example 3

The procedure of Example 1 and materials used in Example 1 were followed and used with the following exceptions. The melt temperature was 190°C and the nip pressure was 5624 grams per square centimeters (80 p.s.i.). The film was "Estane" (TM) polyurethane resin (58309—021, B. F. Goodrich, Cleveland, Ohio). The amount of adhesive utilized was 21 grams per square meter. The results of the tests were as follows:

Average grams per 2.5 cm. width

| | Adhesion to liner | Initial adhesion to skin (6 subjects) | Adhesion to releasable layer |
|---|---|---|---|
| Non-Sterile | 6 | 62.9 | 35.0 |
| Sterile | 7 | 43.6 | 34.0 |

The $F_{10}$ modulus of the film was 113.5 grams (0.25 pound).

Example 4

Styrene butadiene block copolymer resin ("Kraton" 1101 (TM) thermoplastic rubber, Shell Chemical Co., Houston, Texas) was solvent cast into a 12 micron (0.5 mil) film using a 75 microns (3 mil) knife orifice, 23 cm. (9-inch) wide laboratory knife coater. The block copolymer resin was applied as a 25% solid solution in toluene solvent to the silicone release side of a polyester film. The polyester film was 25.4 microns (1 mil) clear "Mylor" (TM) brand polyester which was one-side coated with silicone. (1—1 mil-Mylor-164, Daubert Chemical Co., Oak Brook, Illinois). The solvent was dried off in a 93°C (200°F) oven. The silicone coated polyester film was the releasable layer. The adhesive of Example 1 was applied to the surface of the film not attached to the polyester film by means of laminating a liner containing 21 grams of the adhesive per square meter on the silicone-coated paper referred to in Example 1 by means of dry lamination process between two squeeze rolls on a Laminex Model 12V (TM) machine (Laminex, Inc., Matthews, North Carolina). Test results were as follows:

Average grams per 2.5 cm. width

| | Adhesion to liner | Initial adhesion to skin (1 subject) | Adhesion to releasable layer |
|---|---|---|---|
| Non-Sterile | 5.0 | 48 | 7.5 |

The releasable layer was transparent.

Example 5

The procedure of Example 1 was followed except that a 25 micron (1 mil) "Hytrel" 4056 (TM) brand polyester elastomer (E. I. DuPont de Nemours & Co., Wilmington, Delaware) film was prepared. The adhesive of Example 1 at the same coating weight was utilized. The melt temperature was 204°C and the nip pressure was 5624 grams per square cm (80 p.s.i.). The adhesive was applied as in Example 4. The releasable layer was the clay coated paper of Example 1. The results of the tests were as follows:

Average grams per 2.5 cm. width

| Adhesion to liner | Initial adhesion to skin (one subject) | Adhesion to releasable layer |
|---|---|---|
| 6 | 55 | 45 |

The $F_{10}$ modulus of the film was 363 grams (0.8 pound).

6

# 0 051 935

In all of the above Examples the samples were utilized as described above and placed on the skin. No blocking or significant wrinkling of the film occurred prior to placement on the skin and the skin was not significantly compressed under the film. The films were conformable to the skin. The releasable layer attached more tenaciously to the respective film than the respective adhesive to the liner and the respective film attached more tenaciously to the skin than the respective film to respective releasable layer.

## Claims

1. A medical device (1) for covering a portion of the anatomical surfaces (21) of a living being comprising:
(a) a relatively thin polymeric film (2) having an upper surface and a lower surface, said film being conformable to said portion of said anatomical surfaces (21);
(b) a pressure sensitive adhesive (3) covering at least a portion of said upper surface of said film;
(c) a release liner (4) attached to said adhesive-covered upper surface of said film and;
(d) a releasable layer (7) attached to said lower surface of said film,
characterized in that said releasable layer (7) comprises a frame (8) attached to at least a major portion of the periphery of said lower surface of said film (2) to support said film in a flat, wrinkle-free condition until adhered to said portion of said anatomical surfaces (21), said releasable layer (7) having an opening (9) in the central portion thereof exposing a substantial portion of said film (2) and said releasable layer (7) being attached to said lower surface of said film more tenaciously than the release liner (4) is attached to said adhesive-covered upper surface of said film.

2. The medical device according to claim 1 further characterized in that the portion of said releasable layer (7) which is cut to provide said opening (9) in said frame (8) is retained in said opening until it is removed by the user prior to application of said device to said portion of said anatomical surfaces (21).

3. The medical device according to claim 1 further characterized in that said film permits visual observation of the portion of the anatomical surfaces (21) to which it is adhered.

4. A medical device (1) for covering a portion of the anatomical surfaces (21) of a living being comprising:
(a) a relatively thin polymeric film (2) having an upper surface and a lower surface, said film being conformable to said portion of said anatomical surfaces (21);
(b) a pressure sensitive adhesive (3) covering at least a portion of said upper surface of said film;
(c) a release liner (4) attached to said adhesive-covered upper surface of said film and;
(d) a releasable layer (7) attached to said lower surface of said film,
characterized in that said releasable layer (7) is divided into at least two parts which may be removed from said film independently from each other, at least one of said parts overlying the central portion of said film so that when removed from said film, a major portion of said film is exposed, and at least one of said parts overlying a substantial portion of the periphery of said film so that when the part of said releasable layer overlying the central portion of said film is removed first, the part of said releasable layer overlying the periphery of said film provides a frame to support said film in a flat, wrinkle-free condition until adhered to said portion of said anatomical surfaces (21), and in that said releasable layer (7) is attached more tenaciously to said lower surface of said film than said release liner (4) is attached to said adhesive-covered upper surface of said film.

5. The device according to claim 1 or 4 further characterized in that said film (2) has a $F_{10}$ modulus of 454 grams or less.

6. The device according to claim 4 further characterized in that said film is moisture vapor permeable and selected from the group consisting of polyurethane, elastomeric polyester and blends of polyester and polyurethane.

7. The device of claim 4 wherein at least a portion of said releasable layer (7) is at least translucent to permit visual observation therethrough.

8. The device of claim 7 in which the releasable layer (7) is a translucent film.

9. The device of claim 7 in which the releasable layer (7) is a transparent film.

10. The device of claim 1 which comprises an appliance extending through said opening (9) and attached to the surface of said film opposite to the surface containing said pressure-sensitive adhesive.

11. The device of claim 4 which comprises an absorbent material attached to the adhesive coated surface of said film.

12. The device of claim 4 in the form of a roll in which there is no release liner (4) and the releasable layer (7) serves as the release liner (4).

## Patentansprüche

1. Medizinische Einrichtung (1) zum Abdecken eines Teiles der anatomischen Oberflächen (21) eines Lebewesens, mit
(a) einem relativ dünnen polymeren Film (2), der eine obere und eine untere Fläche besitzt und auf den genannten Teil der genannten anatomischen Flächen (21) passend auflegbar ist,
(b) einem Haftkleber (3), der mindestens einen Teil der oberen Fläche des Films bedeckt;

7

**0 051 935**

(c) einer Antihaftauflage (4), die an der klebstoffbedeckten oberen Fläche des Films angebracht ist, und

(d) einer ablösbaren Lage (7), die an der unteren Fläche des Films abgebracht ist,

dadurch gekennzeichnet, daß die ablösbare Lage (7) einen Rahmen (8) aufweist, der mindestens an dem größten Teil des Umfanges der unteren Fläche des Films (2) angebracht ist und den Film abstützt und in einem ebenen, faltenlosen Zustand hält, bis der Film an dem genannten Teil der anatomischen Flächen (21) angeklebt wird, daß die ablösbare Lage (7) in ihrem mittleren Teile eine Ausnehmung (9) besitzt, durch die hindurch ein beträchtlicher Teil des Films (2) freiliegt, und daß die Verbindung zwischen der ablösbaren Lage (7) und der unteren Fläche des Films reißfester ist als die Verbindung der zwischen Antihaftauflage (4) und der klebstoffbedeckten oberen Fläche des Films.

2. Medizinische Einrichtung nach Anspruch 1, dadurch gekennzeichnet, daß der zur Bildung der Ausnehmung (9) in dem Rahmen (8) ausgeschnittene Teil der ablösbaren Lage (7) in der Öffnung belassen wird, bis er von dem Benutzer entfernt wird, bevor die Einrichtung auf dem genannten Teil der anatomischen Flächen (21) aufgelegt wird.

3. Medizinische Einrichtung nach Anspruch 1, dadurch gekennzeichnet, daß der Film die Betrachtung des mit dem Film verklebten Teils der anatomischen Flächen (21) gestattet.

4. Medizinische Einrichtung (1) zum Abdecken eines Teiles der anatomischen Oberflächen (21) eines Lebewesens, mit

(a) einem relativ dünnen polymeren Film (2), der eine obere und eine untere Fläche besitzt und auf den genannten Teil der genannten anatomischen Flächen (21) passend auflegbar ist,

(b) einem Haftkleber (3), der mindestens einen Teil der oberen Fläche den Films bedeckt,

(c) einer Antihaftauflage (4), die an der klebstoffbedeckten oberen Fläche des Films angebracht ist, und

(d) einer ablösbaren Lage (7), die an der unteren Fläche des Films angebracht ist,

dadurch gekennzeichnet, daß die ablösbare Lage (7) in mindestens zwei Teile geteilt ist, die unabhängig voneinander entfernbar sind und von denen mindestens einer über dem mittleren Bereich des Films liegt, so daß nach einer Abnahme von dem Film der größte Teil desselben freiliegt, und mindestens einer der Teile über einem beträchtlichen Teil des Umfanges des Films liegt, so daß nach der Abnahme zunächst des über dem mittleren Bereich des Films liegenden Teils der ablösbaren Lage der über dem Umfang des Films liegende Teil der ablösbaren Lage einen Rahmen bildet, der den Film abstützt und in einem abenen, faltenlosen Zustand hält, bis der Film an dem genannten Teil der anatomischen Flächen (21) angeklebt wird, und daß die Verbindung zwischen der ablösbaren Lage (7) und der unteren Fläche des Films reißfester ist als die Verbindung der zwischen Antihaftauflage (4) und der klebstoffbedeckten oberen Fläche des Films.

5. Einrichtung nach Anspruch 1 oder 4, dadurch gekennzeichnet, daß der $F_{10}$-Modul des Films (2) 454 g oder weniger beträgt.

6. Einrichtung nach Anspruch 4, dadurch gekennzeichnet, daß der Film feuchtigkeitsdampfdurchlässig ist und daß er aus der Gruppe ausgewählt ist, die aus den Polyurethanen, elastomeren Polyestern und den Gemischen von Polyestern und Polyurethanen besteht.

7. Einrichtung nach Anspruch 4, dadurch gekennzeichnet, daß mindestens ein Teil der ablösbaren Lage (7) mindestens so durchscheinend ist, daß eine Betrachtung durch diese Schicht hindurch möglich ist.

8. Einrichtung nach Anspruch 7, dadurch gekennzeichnet, daß die ablösbare Lage (7) ein durchscheinender Film ist.

9. Einrichtung nach Anspruch 7, dadurch gekennzeichnet, daß die ablösbare Lage (7) ein durchsichtiger Film ist.

10. Einrichtung nach Anspruch 1 mit einem Mittel, das die Ausnehmung (9) durchsetzt und an jener Fläche des Films angebracht ist, die der mit dem Haftkleber versehenen Fläche entgegengesetzt ist.

11. Einrichtung nach Anspruch 4 mit einem absorptionsfähigen Material, das an der klebstoffüberzogenen Fläche des Films angebracht ist.

12. Einrichtung nach Anspruch 4 in Form einer Rolle, dadurch gekennzeichnet, daß keine Antihaftauflage (4) vorgesehen ist und die ablösbare Lage (7) als Antihaftauflage (4) dient.

**Revendications**

1. Dispositif médical (1) pour couvrir une portion des surfaces anatomiques (21) d'un organisme vivant comprenant:

(a) une pellicule polymère (2) relativement mince ayant une surface supérieure et une surface inférieure, ladite pellicule étant adaptable à ladite portion desdites surfaces anatomiques (21);

(b) un adhésif autocollant (3) couvrant au moins une portion de ladite surfaace supérieure de ladite pellicule;

(c) une doublure de séparation (4) fixée à ladite surface supérieure recouverte d'adhésif de ladite pellicule; et

(d) une couche amovible (7) fixée à ladite surface supérieure de ladite pellicule,

caractérisé en ce que ladite couche amovible (7) comprend un cadre (8) fixé à au moins une portion majeure du pourtour de ladite surface inférieure de ladite pellicule (2) pour maintenir ladite pellicule sous une forme plane et sans pli jusqu'à l'adhérence avec ladite portion desdites surfaces anatomiques (21), ladite couche amovible (7) ayant une ouverture (9) dans sa portion centrale faisant apparaître une portion

8

notable de ladite pellicule (2) et ladite couche amovible (7) étant fixée à ladite surface inférieure de ladite pellicule de façon plus tenace que la doublure de séparation (4) n'est fixée à ladite surface supérieure recouverte d'adhésif de ladite pellicule.

2. Dispositif médical selon la revendication 1, caractérisé de plus en ce que la portion de ladite couche amovible (7) qui est découpée pour ménager ladite ouverture (9) dans ledit cadre (8) est retenue dans ladite ouverture jusqu'à ce qu'elle soit retirée par l'utilisateur avant l'application dudit dispositif à ladite portion desdites surfaces anatomiques (21).

3. Dispositif médical selon la revendication 1, caractérisé de plus en ce que ladite pellicule permet l'observation visuelle de la portion des surfaces anatomiques (21) à laquelle elle adhère.

4. Dispositif médical (1) pour recouvrir une portion des surfaces anatomiques (21) d'un organisme vivant comprenant:

(a) une pellicule polymère relativement mince (2) ayant une surface supérieure et une surface inférieure, ladite pellicule étant adaptable à ladite portion desdites surfaces anatomiques (21),

(b) un adhésif autocollant (3) couvrant au moins une portion de ladite surface supérieure de ladite pellicule,

(c) une doublure amovible (4) fixée à ladite surface supérieure recouverte d'adhésif de ladite pellicule; et

(d) une couche amovible (7) fixée à ladite surface inférieure de ladite pellicule,

caractérisé en ce que ladite couche amovible (7) est divisée en au moins deux parties qui peuvent être séparées de ladite pellicule indépendamment l'une de l'autre, au moins l'une desdites parties recouvrant la portion centrale de ladite pellicule de façon à ce que lorsqu'elle est séparée de ladite pellicule une portion majeure de ladite pellicule soit apparente, et au moins une desdites parties recouvrant une portion notable du pourtour de ladite pellicule pour que, lorsque la partie de ladite couche amovible recouvrant la portion centrale de ladite pellicule est d'abord retirée, la partie de ladite couche amovible recouvrant le pourtour de ladite pellicule fournisse un cadre pour maintenir ladite pellicule en un état plan et sans pli jusqu'à ce qu'elle ait adhéré à ladite portion desdites surfaces anatomiques (21), et en ce que ladite couche amovible (7) est fixée de façon plus tenace à ladite surface inférieure de ladite pellicule que ladite doublure amovible (4) n'est fixée à ladite surface supérieure recouverte d'adhésif de ladite pellicule.

5. Dispositif selon la revendication 1 ou 4, caractérisé de plus en ce que ladite pellicule (2) a un module $F_{10}$ de 454 g ou moins.

6. Dispositif selon la revendication 4, caractérisé de plus en ce que ladite pellicule est perméable à la vapeur d'eau et est choisie parmi le groupe constitué par les polyuréthane, polyester élastomère et mélanges de polyester et de polyuréthane.

7. Dispositif selon la revendication 4, dans lequel au moins une portion de ladite couche amovible (7) est au moins translucide pour permettre l'observation visuelle à travers elle.

8. Dispositif selon la revendication 7, dans lequel la couche amovible (7) est une pellicule translucide.

9. Dispositif selon la revendication 7 dans lequel la couche amovible (7) est une pellicule transparente.

10. Dispositif selon la revendication 1 comprenant un article s'étendant à travers ladite ouverture (9) et fixé à la surface de ladite pellicule opposée à la surface contenant ledit adhésif autocollant.

11. Dispositif selon la revendication 4 comprenant une matière absorbante fixée à la surface revêtue d'adhésif de ladite pellicule.

12. Dispositif selon la revendication 4, sous forme d'un rouleau dans lequel il n'y a pas de doublure de séparation (4) et la couche amovible (7) sert de doublure de séparation (4).

FIG.1

FIG.2

FIG.3

FIG.4

FIG.5